# EUROPEAN PATENT APPLICATION

(11) **EP 4 174 085 A1**
(43) Date of publication of application: **03.05.2023**
(21) Application number: 21834296.2
(22) Date of filing: 28.06.2021
(51) Int. Cl.: C07K 16/10, C12N 15/13, C12N 15/50, G01N 33/53, G01N 33/543

(54) **ANTIBODY AGAINST SARS-COV-2, METHOD FOR DETECTING SARS-COV-2 USING ANTIBODY AND KIT CONTAINING ANTIBODY**

(30) Priority: 29.06.2020 JP 2020111984
(71) Applicant: Public University Corporation Yokohama City University, Yokohama-shi, Kanagawa 236-0027 (JP); Kanto Kagaku Kabushiki Kaisha, Tokyo 103-0023 (JP)
(72) Inventor: RYO, Akihide, Yokohama-shi, Kanagawa 236-0004 (JP); YAMAOKA, Yutaro, Isehara-shi, Kanagawa 259-1146 (JP); KIKUCHI, Sayaka, Isehara-shi, Kanagawa 259-1146 (JP)
(74) Representative: Meissner Bolte Partnerschaft mbB
(86) International application number: PCT/JP2021/024267
(87) International publication number: WO 2022/004622

(57) **Abstract**

The present invention addresses the problem of providing a means for detecting a constituting protein of nucleocapsid derived from SARS-CoV-2. This problem has been solved by providing an antibody binding to a constituting protein of nucleocapsid derived from SARS-CoV-2 or a fragment of the antibody, a method for detecting a constituting protein of the SARS-CoV-2-derived nucleocapsid with the use of the antibody or a fragment thereof, a kit for detecting a constituting protein of the SARS-CoV-2-derived nucleocapsid, said kit containing the antibody or a fragment thereof, etc.

## Description

### [Technical Field]

The present invention relates to an antibody against SARS-CoV-2, a method for detecting SARS-CoV-2 using said antibody, and a kit comprising said antibody and the like.

### [Background Art]

2019 Novel Coronavirus (hereinafter referred to as "SARS-CoV-2" in some cases) is a coronavirus belonging to the SARS-associated coronaviruses, which was first identified its outbreak near Wuhan, China in 2019. It is pathogenic to humans and causes novel coronavirus infection (COVID-19).

Currently, WHO has shown the recognition that the spread of SARS-CoV-2 is currently equivalent to a pandemic (global epidemic). On the other hand, no treatment has been established for patients infected with SARS-CoV-2. Therefore, in order to take appropriate measures to prevent the spread of infection, it is extremely important to find patients infected with SARS-CoV-2 by rapid testing as well as to investigate the rate of infection, or route of infection by checking the antibody prevalence in serum using immunological tests.

As detection methods for SARS-CoV-2, genetic tests such as real-time reverse transcription-PCR using collected nasal swab, pharyngeal swab, or sputum as specimens (Non-Patent Documents 1 and 2) are mainly used. By this, it is possible to determine the presence or absence of SARS-CoV-2 infection. On the other hand, by the aforesaid genetic test, there happened many cases in which SARS-CoV-2 is not detected in aforesaid specimens (false negative) due to the influence by the location of collection, timing of collection, and storage method of specimen, even though the specimens are infected with SARS-CoV-2, and this is one of the reasons of the spread of SARS-CoV-2 infection. Also, genetic testing requires a special device that detects fluorescence in real time during PCR, and it takes several hours to obtain the results, so it remains a problem from the viewpoint of early detection in places such as infection sites, quarantine sites, and clinical sites, where the facility is not sufficient. Thus, immunological tests using antigen-antibody reactions against SARS-CoV-2 derived proteins are required as tests that combine accuracy, speed, and convenience.

The spike (hereinafter referred to as "S" in some cases) protein, which is present on the surface of the SARS-CoV-2 envelope and is involved in infection of host cells, is known as a protein derived from SARS-CoV-2 (Non-Patent Document 3). However, the SARS-CoV-2 derived S protein is a glycoprotein, which is made up of bound many sugar chains (Non-Patent Document 3), and it is considered extremely difficult to artificially prepare a protein that exactly reproduces its structure using chemical synthesis or genetically engineered microorganisms. The S protein derived from the SARS coronavirus, which is genetically related to SARS-CoV-2, is known to be easily mutated its amino acid sequence as the SARS coronavirus replicates (Non-Patent Document 4). Likewise, SARS-CoV-2 derived S protein is considered to be easily mutated in its amino acid sequence as SARS-CoV-2 replicates. For this reason, antibodies prepared against the SARS-CoV-2 derived S protein may not show antigen-antibody reactions against mutants of the S protein, and thus, there is a risk that immunological tests using antibodies against the SARS-CoV-2 derived S protein may produce false negative test results depending on the specimens.

On the other hand, a protein constituting SARS-CoV-2 derived nucleocapsid (hereinafter referred to as "NP" in some cases) is also known as a SARS-CoV-2 derived protein. The NP derived from the SARS coronavirus, which is genetically related to SARS-CoV-2, is known to be the most abundant among the various SARS coronavirus-derived proteins produced by infection (Non-Patent Document 5), and likewise, SARS-CoV-2 derived NPs are thought to be the most abundant of the various SARS-CoV-2 derived proteins produced by infection. For this reason, SARS-CoV-2 derived NPs are expected to be relatively easy to detect from specimens. In fact, a case of a preparation of antibody against a partial peptide of SARS-CoV-2 derived NP has already been reported (Non-Patent Document 6). However, the antibodies prepared in said case shows the antigen-antibody reaction against NPs derived from MERS coronavirus and SARS coronavirus, and does not have a specificity for the SARS-CoV-2 derived NP (Non-Patent Document 6). Hence, in immunological tests using said antibody, there is a risk of producing false positive test results depending on the specimen.

### [Citation List]

### [Non-Patent Documents]

[Non-Patent Document 1] 2019-Specimen Collection and Transport Manual for Patients Suspected of nCoV (novel coronavirus) Infection (National Institute of Infectious Diseases, April 16, 2020)
[Non-Patent Document 2] Pathogen Detection Manual 2019-nCoV Ver. 2.9.1 (National Institute of Infectious Diseases, March 19, 2020)
[Non-Patent Document 3] Chen X., et al., Cell. Mol. Immunol.,
(2020) doi: https://doi.org/10.1038/s41423-020-0426-7
[Non-Patent Document 4] Qin E., et al., Geno., Prot. Bioinfo., 1(2), 101-107 (2003)
[Non-Patent Document 5] Wang J., et al., Geno., Prot. Bioinfo., 1(2), 145-154 (2003)
[Non-Patent Document 6] Li M., et al., medRxiv, (2020) doi: https://doi.org/10.1101/2020.02.20.20025999

### [Summary of Invention]

### [Problems to Be Solved by Invention]

The present invention is aim to provide a means of detecting proteins constituting SARS-CoV-2 derived nucleocapsid.

### [Means of Solving Problems]

The present inventors analyzed the SARS-CoV-2 derived NP and other coronavirus-derived NPs. As a result, the present inventors found a region of low identity between the amino acid sequence of SARS-CoV-2 derived NP and other coronavirus-derived NPs. Then in the light of the above-mentioned problem, the inventors prepared fragments of SARS-CoV-2 derived NP corresponding to said region and came to complete the present invention by using these as immunogens and preparing antibodies according to the usual method.

That is to say, the present invention relates to the following:
[1] An antibody or fragment thereof which binds to a protein constituting a SARS-CoV-2 derived nucleocapsid, wherein an epitope which is recognized by said antibody or fragment thereof is located at positions 121 to 419 of the amino acid sequence of SEQ ID NO:1.
[2] The antibody or fragment thereof according to [1], wherein the epitope is located at positions 208 to 222, positions 332 to 351, positions 374 to 397, or positions 398 to 419 of the amino acid sequence of SEQ ID NO:1.
[3] The antibody or fragment thereof according to [1] or [2], wherein the antibody or fragment thereof specifically binds to a protein constituting a SARS-CoV-2 derived nucleocapsid.
[4] The antibody or fragment thereof according to any one of [1]-[3], wherein the antibody is a monoclonal antibody.
[5] The antibody or fragment thereof according to any one of [1]-[4], wherein the fragment is a Fab fragment, Fab/c fragment, Fv fragment, Fab' fragment or F(ab')2 fragment.
[6] A method for detecting a protein constituting a SARS-CoV-2 derived nucleocapsid within a sample, wherein the method comprises a step of contacting the sample with an antibody or fragment thereof according to any one of [1]-[5].
[7] The method according to [6], wherein the sample is a biological sample.
[8] The method according to [6] or [7], wherein the method further comprises a step of detecting a protein constituting a SARS-CoV-2 derived nucleocapsid by at least one immunological test selected from the group consisting of ELISA, immunochromatography and filter antigen assay.
[9] A kit for detecting a protein constituting a SARS-CoV-2 derived nucleocapsid, comprising an antibody or fragment thereof according to any one of [1]-[5].
[10] The kit according to [9], wherein the kit is a form of immunochromatographic strip.

### [Advantageous Effects of Invention]

The presence or absence of SARS-CoV-2 infection, or the past history can be detected with accuracy, speed, and convenience by an antibody or fragment thereof that specifically bind to SARS-CoV-2 derived NPs, a method for detecting SARS-CoV-2 derived NPs using said antibody or fragment thereof, and a kit for detecting a protein constituting SARS-CoV-2 derived nucleocapsid comprising said antibody or fragment thereof and the like of the present invention. That is to say, by the present invention, an immunological test equipped with high practicality in the early detection in places such as infection sites, quarantine sites, and clinical sites, where the facility is not sufficient, can be provided. This is because the epitope which is recognized by the antibody of the present invention or fragment thereof that specifically bind to SARS-CoV-2 derived NPs constitutes a region of low identity between the amino acid sequence of SARS-CoV-2 derived NP and other coronavirus-derived NPs and it is possible to specifically detect SARS-CoV-2 derived NPs from the specimen.

Also, the method of detecting SARS-CoV-2 derived NPs with the antibody of the present invention or fragment thereof that specifically bind to SARS-CoV-2 derived NPs can reduce the number of cases where false positive and false negative occur.

### [Brief description of the drawings]

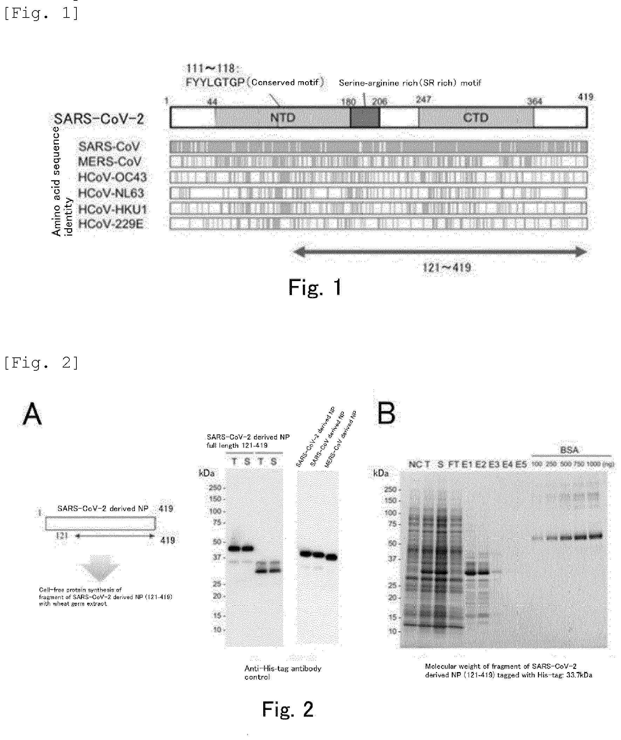

[Fig. 1] Figure 1 shows the sequence identities between SARS-CoV-2 derived NPs and NPs derived from other coronaviruses (SARS, MERS, OC43, and MERS, OC43, and NL63, HKU1, and 229E). Fragments of SARS-CoV-2 derived NPs, excluding motifs that are highly antigenic and shared with NPs derived from other coronaviruses (region 121 to 419 of the amino acid sequence represented by SEQ ID NO:1) are used as antigens to prepare antibodies.
[Fig. 2] Figure 2 shows the results of the preparation of fragments of SARS-CoV-2 derived NPs. Figure 2A shows the results in which the expression of fragments of SARS-CoV-2 derived NPs were confirmed. Figure 2B shows the results in which fragments of SARS-CoV-2 derived NPs were purified.

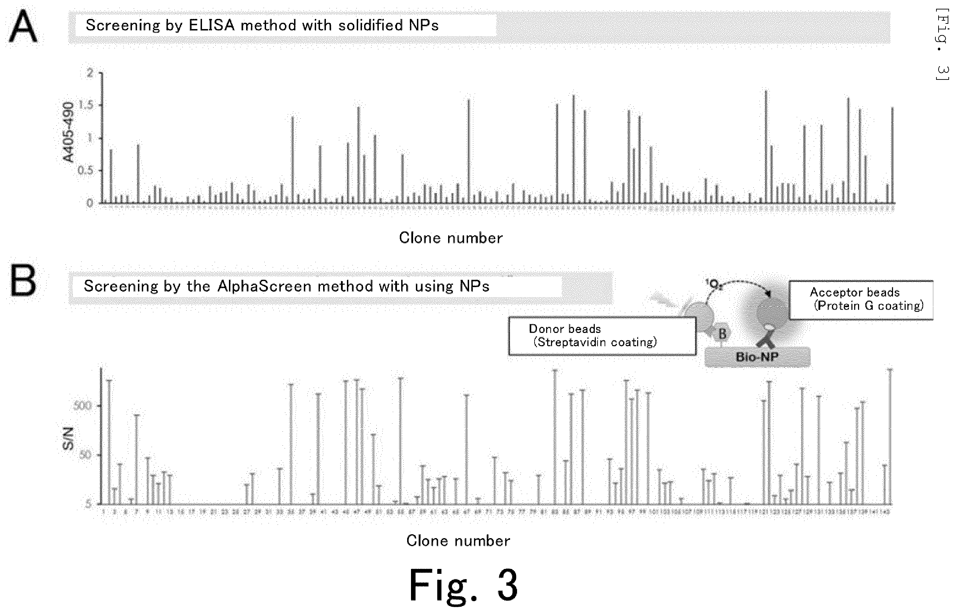

[Fig. 3] Figure 3 shows the results of screening of hybridoma cells producing monoclonal antibodies, which are highly reactive to fragments of SARS-CoV-2 derived NPs. Figure 3A shows the results of screening by ELISA method with solidified fragments of SARS-CoV-2 derived NPs. Figure 3B shows the results of screening by the AlphaScreen method using fragments of SARS-CoV-2 derived NPs.

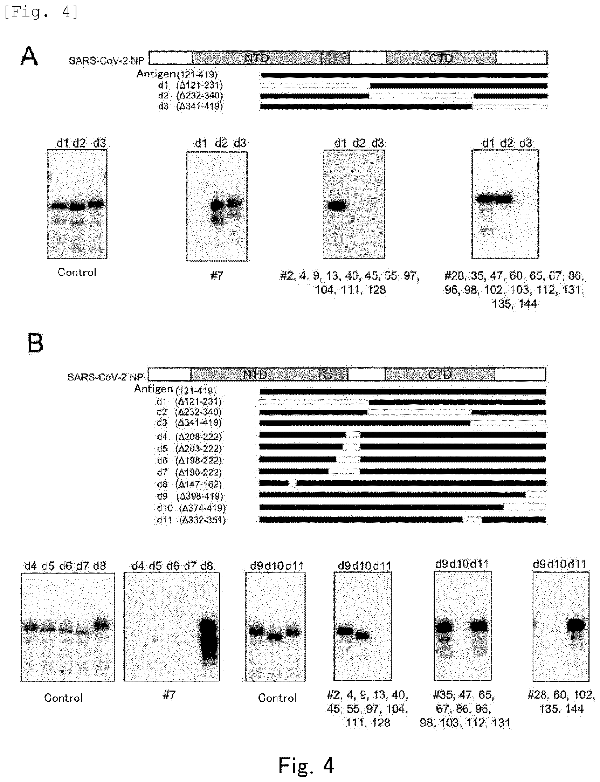

[Fig. 4] Figure 4 shows the results of epitope analysis of each monoclonal antibody prepared in Example 2. Figure 4A shows the results of the study of epitopes recognized by each monoclonal antibody by preparing three kinds of recombinant proteins (deletion mutants) d1-d3, respectively lacking each region of the amino acid sequence of a fragment of SARS-CoV-2 derived NPs (upper schematic diagram) and comparing their reactivity (Western blot results in the lower column). Figure 4B shows the results of the study of epitopes recognized by each monoclonal antibody by preparing 8 kinds of recombinant proteins (deletion mutants) d4-d11 respectively lacking each region of the amino acid sequence of a fragment of SARS-CoV-2 derived NPs (upper schematic diagram) and comparing their reactivity (Western blot results in the lower column).

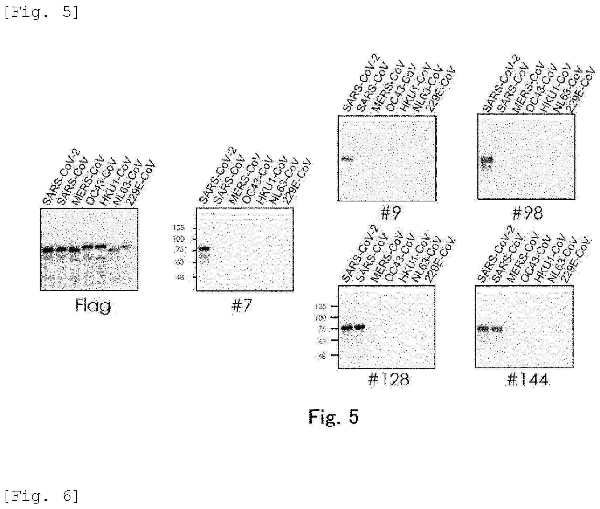

[Fig. 5] Figure 5 shows the results of the specificity analysis of each monoclonal antibody prepared in Example 2. Using each of NPs derived from other human coronaviruses (SARS, MERS, OC43, NL63, HKU1, and 229E), the specificity of each monoclonal antibody was analyzed by Western blotting.

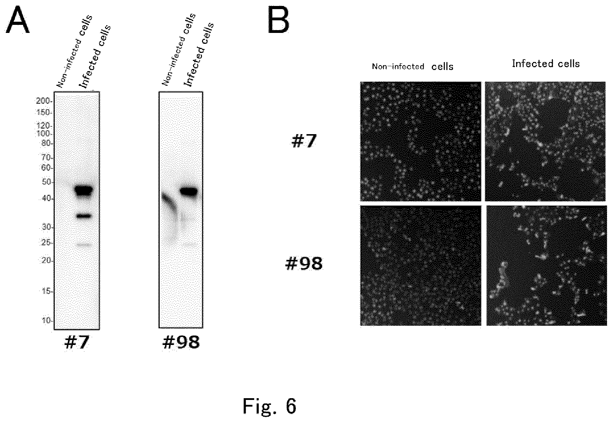

[Fig. 6] Figure 6 shows the results of immunostaining of SARS-CoV-2-infected cells. Figure 6A shows the expression of SARS-CoV-2 derived NPs in aforesaid infected cells. Figure 6B shows the results where aforesaid infected cells were immunostained with monoclonal antibodies (#7, #98) prepared in Example 2.

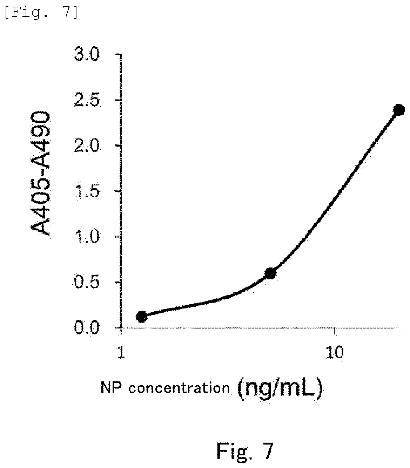

[Fig. 7] Figure 7 shows the results of SARS-CoV-2 derived NP detection by ELISA using the monoclonal antibodies (#9 and #98) prepared in Example 2.

### [Embodiments for Carrying out Invention]

Unless otherwise defined herein, all technical and scientific terms used herein have similar meanings as those usually understood by those skilled in the art. All patents, applications, and other publications and information referred to herein are incorporated herein by reference in their entirety.

### [An antibody or fragment thereof which binds to a protein constituting a SARS-CoV-2 derived nucleocapsid]

An aspect of present invention relates to an antibody or fragment thereof which binds to a protein constituting a SARS-CoV-2 derived nucleocapsid, wherein an epitope which is recognized by said antibody or fragment thereof is located at positions 121 to 419 of the amino acid sequence of SEQ ID NO:1 (hereinafter referred to as "an antibody of the present invention or fragment thereof" in some cases).

In one embodiment, the epitope recognized by the antibody of the present invention or fragment thereof is located at positions 208 to 222, positions 332 to 351, positions 374 to 397, or positions 398 to 419 of the amino acid sequence of SEQ ID NO:1. In one embodiment, the antibody of the present invention or fragment thereof specifically binds to a protein constituting a SARS-CoV-2 derived nucleocapsid.

In the present invention, "SARS-CoV-2" refers to a coronavirus belonging to the SARS-associated coronaviruses, which was first identified its outbreak near Wuhan, China in 2019 and used interchangeably with "2019 Novel Coronavirus". SARS-CoV-2 is pathogenic to humans and causes acute respiratory disease (COVID-19). SARS-CoV-2, similarly to other coronaviruses, is constituted of a spike, nucleocapsid, endogenous membrane protein, envelope protein, and RNA. Among those, nucleocapsid binds to RNA to form NPs, and it is surrounded by spike, intrinsic membrane protein, and envelope protein which are bound to lipid to form the envelope. The genome sequence of SARS-CoV-2 is published under the GenBank accession number MN908947.3.

In the present invention, "a protein constituting of nucleocapsid" refers to a protein that constitutes a complex consisting of nucleocapsid and RNA, and is used interchangeably with "NP". The amino acid sequence of SARS-CoV-2 derived NP is represented by SEQ ID NO:1.

In the present invention, "a fragment of SARS-CoV-2 derived NPs" refers to a fragment of protein consisting of a region of the amino acid sequence in SARS-CoV-2 derived NPs (SEQ ID NO:1) that consists of amino acid sequence having low identity between the amino acid sequence of SARS-CoV-2 derived NP and the amino acid sequence of NPs derived from other coronaviruses (region 121 to 419 of the amino acid sequence represented by SEQ ID NO:1). SARS-CoV-2 derived NP fragments have 90% or more sequence identity to the amino acid sequence represented by SEQ ID NO:2. From the viewpoint of accurate, simple, and rapid detection of SARS-CoV-2 derived NPs, fragments of SARS-CoV-2 derived NPs preferably have a sequence identity of 95% or more to the amino acid sequence represented by SEQ ID NO:2, especially preferably have a sequence identity of 98% or more, even more preferably a sequence identity of 100%. In one embodiment, the fragment of the SARS-CoV-2 derived NPs consists of the amino acid sequence represented by SEQ ID NO:2. Since the fragment of SARS-CoV-2 derived NPs corresponds to a region of low identity between the amino acid sequence of SARS-CoV-2 derived NP and the amino acid sequences of NPs from other coronaviruses, it is possible to prepare said fragment and to obtain the antibody of the present invention or fragment thereof by using this as an antigen according to the usual methods.

The antibody of the present invention is, for example, a complete immunoglobulin molecule, is preferably IgM, IgD, IgE, IgA or IgG, and is more preferably IgG1, IgG2a, IgG2b, IgG3 or IgG4. Also, the antibody of the present invention can be a modified and/or altered antibody, such as chimeric and humanized antibodies. Also, the antibody of the present invention can be a monoclonal or polyclonal antibody, a modified or altered monoclonal or polyclonal antibody, or a recombinantly or synthetically prepared or synthesized antibody. From the viewpoint of accurate, simple, and rapid detection of SARS-CoV-2 derived NPs, it is preferred that the antibody is a monoclonal antibody.

A fragment of the antibody of the present invention can be a portion of immunoglobulin molecules, such as a Fab fragment, VL-, VH- or CDR-regions, but the fragment of the antibody of the present invention can specifically recognize SARS-CoV-2 derived NPs to the similar extent as the antibody of the present invention. The fragment of the antibody of the present invention is an antibody fragment, as well as a portion of those, such as a separated light and heavy chain, Fab, Fab/c, Fv, Fab', F(ab')2. In one embodiment, the fragment of the antibody of the present invention is a Fab fragment, Fab/c fragment, Fv fragment, Fab' fragment or F(ab')2 fragment.

Also, the antibody of the present invention or fragment thereof can be an antibody derivative such as a bifunctional antibody, or antibody construct such as single chain Fv (scFv), bispecific scFv or an antibody fusion protein.

### [A method for detecting proteins constituting SARS-CoV-2 derived nucleocapsid within samples]

Another aspect of the present invention relates to a method for detecting a protein constituting a SARS-CoV-2 derived nucleocapsid within a sample, wherein the method comprises a step of contacting the sample with the antibody of the present invention or fragment thereof (hereinafter referred to as "a method of the present invention" in some cases). In one embodiment, in the method of present invention, the sample is a biological sample. In one embodiment, in the method of present invention further comprises a step of detecting a protein constituting a SARS-CoV-2 derived nucleocapsid by at least one immunological test selected from the group consisting of ELISA, immunochromatography and filter antigen assay.

In one embodiment, the method of the present invention comprises the step of performing an antigen-antibody reaction between the antibody of the present invention or fragment thereof and SARS-CoV-2 derived NPs. In one embodiment, the method of the present invention comprises a plurality of the steps of performing an antigen-antibody reaction between the antibody of the present invention or fragment thereof and SARS-CoV-2 derived NPs. In one embodiment, the antibody of the present invention or fragment thereof is different in its epitope at each and every time of the step. In one embodiment, the antibody of the present invention or fragment thereof is labeled with a labeling substance. For example, the method of the present invention comprises the step of performing an antigen-antibody reaction between the antibody of the present invention or fragment thereof that is unlabeled and SARS-CoV-2 derived NPs, and the step of performing an antigen-antibody reaction between the antibody of the present invention or fragment thereof that is labeled by a labeled substance (that is different in its epitope from aforesaid antibody of the present invention or fragment thereof that is unlabeled) and SARS-CoV-2 derived NPs.

In one embodiment, the method of the present invention comprises the step of performing an antigen-antibody reaction between the antibody of the present invention or fragment thereof and SARS-CoV-2 derived NPs, and/or further comprises the step of performing an antigen-antibody reaction between the antibody of the present invention or fragment thereof and an antibody or fragment thereof that recognizes said antibody or fragment thereof. In one embodiment, the antibody of the present invention or fragment thereof, and/or an antibody of fragment thereof that recognizes said antibody or fragment thereof is labeled with a labeling substance.

In the present invention, a "sample" is any sample that can comprise SARS-CoV-2 derived NPs, and is not particularly limited, but for example, biological samples can be listed. In the present invention, a "sample" is used interchangeably with a "specimen".

In the present invention, a "biological sample" is any sample that can comprise SARS-CoV-2 derived NPs, such as human or animal blood, plasma, serum, urine, semen, spinal fluid, saliva, oral mucosa, nasal fluid, sweat, tears, ascites, amniotic fluid, feces, organs such as blood vessels or liver, tissues, cells, or extracts thereof. Preferably, these are cells and secretion fluids which are easy to collect, from such as the oral cavity, tonsils, nasal cavity, pharynx, larynx, trachea, bronchi, and lungs, as well as such as nasal swab, pharyngeal swab, gargle, sputum, tracheal aspirate, bronchoalveolar lavage fluid. The method for collecting these specimens is not particularly limited, and commonly known methods can be employed. Specifically, the method using cotton swabs is often used. Animals are not particularly limited if it can be infected with SARS-CoV-2, including such as dogs and cats.

Biological samples can be collected according to the usual methods.

For example, as described in Non-Patent Document 1, 1 to 2 ml of the serum after being separated is placed in a plastic tube that can be tightly stoppered, and after being covered with a lid, the tube is sealed with Parafilm. The tube may contain a coagulant, and when a blood collection tube containing a serum separator is used, after 1 to 2 ml of serum after centrifugation is transferred into a plastic tube (preferably a sterile tube), after that, it is covered with a lid, and then sealed with Parafilm.

For example, whole blood is collected in a blood collection tube containing an anticoagulant (EDTA-Na or K), as described in Non-Patent Document 1, and 1 to 2 ml is dispensed into a plastic tube that can be tightly stoppered, and after being covered with a lid, it is sealed with Parafilm. If possible, blood corpuscles are separated and peripheral blood mononuclear cells are suspended in cell preservation solution and cryopreserved. The separation of peripheral blood mononuclear cells becomes simple by using BD Vacutainer (registered trademark) CPT (trademark) blood collection tubes for mononuclear cell separation. Also, when aliquoting or blood cell separation is not possible after blood collection, PAXgene (registered trademark) RNA collection tubes may be used for blood collection and stored frozen as is.

For example, lower airway-derived fluid is collected from sputum when sputum is produced, as described in Non-Patent Document 1. When the patient is under ventilator control, tracheal aspirate is collected using a sterilized catheter under aseptic manipulation. When it is not clinically contraindicated, the collection of bronchoalveolar lavage fluid is also to be considered. The collected sputum or aspirate should be placed in a plastic tube with a screw cap, covered with a lid and sealed with Parafilm.

For example, for nasopharyngeal swab solution, as described in Non-Patent Document 1, a sterile cotton swab (such as a flock swab or a cotton swab containing rayon or polyester in its material. A slim one for nasal use) is inserted into the nasal cavity and the nasopharynx is thoroughly swabbed, and the swab is placed in a sterile spit tube containing 1 to 3 ml of viral transport solution (VTM/UTM), cover with a lid, and seal with Parafilm. When no viral transport solution is available, PBS or saline solution is used.

For example, for urine, as described in Non-Patent Document 1, 1 to 2 ml is collected in a test tube (e.g. Falcon tube), covered with a lid, and sealed with Parafilm.

For example, for feces, as described in Non-Patent Document 1, about 0.1 g (the size of a small pea) is collected in a plastic tube that can be tightly stoppered, and is covered with a lid, and then sealed with Parafilm.

In the present invention, "labeled substance" refers to any substance that labels the antibody of the present invention or fragment thereof and/or the antibody or fragment thereof that recognizes said antibody or fragment thereof, and is not particularly limited, as long as those can detect antigen-antibody reactions. From the viewpoint of accurate, simple, and rapid detection of SARS-CoV-2 derived NPs, it is preferred that the labeling substance is one or more selected from enzymes, pigments, metal colloidal particles, latex particles, cellulose particles.

In the present invention, "an antibody of fragment thereof that recognizes the antibody of the present invention or fragment thereof" refers to an antibody of fragment thereof that can specifically bind to the antibody of the present invention or fragment thereof. From the viewpoint of accurate, simple, and rapid detection of SARS-CoV-2 derived NPs, an antibody that recognizes the antibody of the present invention or fragment thereof is preferably one or more selected from a group consisting of IgG, IgM, IgA, IgD and IgE, is especially preferably IgG and/or IgM, and is even more preferably IgG. Also, the antibody that recognizes the antibody of the present invention or fragment thereof can be a modified and/or altered antibody, such as chimeric and humanized antibodies. Also, the antibody that recognizes the antibody of the present invention or fragment thereof can be a monoclonal or polyclonal antibody, a modified or altered monoclonal or polyclonal antibody, or a recombinantly or synthetically prepared or synthesized antibody.

The fragment of the antibody that recognizes the antibody of the present invention or fragment thereof can be a portion of such immunoglobulin molecules, such as a Fab fragment, VL-, VH- or CDR-regions, but the fragment of the antibody that recognizes the antibody of the present invention or fragment thereof, too, can specifically recognize SARS-CoV-2 derived NPs to the similar extent as the antibody that recognizes the antibody of the present invention or fragment thereof. The fragment of the antibody that recognizes the antibody of the present invention or fragment thereof can be an antibody fragment, as well as a separated light and heavy chain, a portion of those such as Fab, Fab/c, Fv, Fab', F(ab')2. In one embodiment, the fragment of the antibody that recognizes the fragment of the antibody of the present invention is a Fab fragment, Fab/c fragment, Fv fragment, Fab' fragment or F(ab')2 fragment.

Also, the fragment of the antibody that recognizes the antibody of the present invention or fragment thereof can be an antibody derivative such as a bifunctional antibody, or antibody construct such as single chain Fv (scFv), bispecific scFv or an antibody fusion protein.

In the present invention, SARS-CoV-2 derived NPs are detected by any immunological test that can detect antigen-antibody reactions between the antibody of the invention or fragment thereof and SARS-CoV-2 derived NPs. The immunological tests include, for example, such as enzyme immunoassay (ELISA, EIA), fluorescence immunoassay (FIA), radioimmunoassay (RIA), luminescence immunoassay (LIA), enzyme antibody assay, fluorescence antibody assay, immunochromatography (immunochromatographic assay), filter antigen assay, immunoturbidimetry, latex turbidimetry, latex agglutination assay, erythrocyte agglutination assay, or particle agglutination assay. From the viewpoint of accurate, simple, and rapid detection of SARS-CoV-2 derived NPs, it is preferred that SARS-CoV-2-derived NPs are detected by ELISA and/or immunochromatography. Also, the SARS-CoV-2-derived NPs contained in a sample can be quantified by creating a calibration curve from the antigen-antibody reaction between a predetermined amount of the antibody of the present invention or fragment thereof and the SARS-CoV-2 derived NPs, and interpolating the measured values into the calibration curve.

### [A kit for detecting a protein constituting a SARS-CoV-2 derived nucleocapsid]

Another aspect of the present invention relates to a kit for detecting a protein constituting a SARS-CoV-2 derived nucleocapsid, comprising the antibody of the present invention or fragment thereof (hereinafter referred to as "a kit for the present invention" in some cases). From the viewpoint of accurate, simple, and rapid detection of SARS-CoV-2 derived NPs, from the viewpoint of detection of SARS-CoV-2-derived NPs by healthcare workers in places such as infection sites, quarantine sites, and clinical sites, where the facility is not sufficient, from the viewpoint of studying epidemiology, it is preferred that the kit of the present invention is a form of immunochromatographic strips.

For example, an immunochromatographic strip is one that has the antibody of the present invention or fragment thereof and an anti-mouse immunoglobulin antibody immobilized at separate sites respectively, i.e., a test line and a control line. In the sample pad of the immunochromatographic strip, the antibody of the present invention or fragments thereof labeled with such as gold colloidal particles, latex particles, fluorescent particles, magnetic particles, is immersed beforehand. The sample dropped onto the sample pad is expanded by capillary action, and when it reaches the test line, only the SARS-CoV-2-derived NPs in the sample react, and when it reaches the control line, only the antibody of the present invention or fragments thereof labeled with gold colloid react. Other compounds in the sample will migrate to the absorbent pad without reacting. By measuring such as the color intensity, reflectance intensity, absorbance, fluorescence intensity, or magnetic intensity of the test and control lines with an immunochromatographic reader, not only the SARS-CoV-2-derived NPs in the sample can be detected but also SARS-CoV-2 derived NPs in a sample can be quantified.

### [A composition for the detection of proteins constituting SARS-CoV-2 derived nucleocapsids]

Another aspect of the present invention relates to a composition for detecting a protein constituting a SARS-CoV-2 derived nucleocapsid, comprising the antibody of the present invention or fragment thereof (hereinafter referred to as "a composition of the present invention" in some cases).

### [A method for diagnosing SARS-CoV-2 infection]

Another aspect of the present invention, relates to a method for diagnosing SARS-CoV-2 infection, wherein the method comprises a step of contacting the sample with an antibody or fragment thereof (hereinafter referred to as "a method for diagnosing of the present invention" in some cases). In one embodiment, the method for diagnosing of the present invention further comprises a step of detecting a protein constituting a SARS-CoV-2 derived nucleocapsid by at least one immunological test selected from the group consisting of ELISA, immunochromatography and filter antigen assay.

In one embodiment, the method for diagnosing of the present invention comprises the step of performing an antigen-antibody reaction between the antibody of the present invention or fragment thereof and SARS-CoV-2 derived NPs. In one embodiment, the method for diagnosing of the present invention comprises a plurality of the steps of performing an antigen-antibody reaction between the antibody of the present invention or fragment thereof and SARS-CoV-2 derived NPs. In one embodiment, the antibody of the present invention or fragment thereof is different in its epitope at each and every time of said step. In one embodiment, the antibody of the present invention or fragment thereof is labeled with a labeling substance. For example, the method for diagnosing of the present invention comprises the step of performing an antigen-antibody reaction between the antibody of the present invention or fragment thereof that is unlabeled and SARS-CoV-2 derived NPs, and the step of performing an antigen-antibody reaction between the antibody of the present invention or fragment thereof that is labeled by a labeled substance (that differs from aforesaid antibody of the present invention or fragment thereof that is unlabeled in its epitope) and SARS-CoV-2 derived NPs.

In one embodiment, the method for diagnosing of the present invention comprises the step of performing an antigen-antibody reaction between the antibody of the present invention or fragment thereof and SARS-CoV-2 derived NPs, and/or further comprises the step of performing an antigen-antibody reaction between the antibody of the present invention or fragment thereof and an antibody or fragment thereof that recognizes said antibody or fragment thereof. In one embodiment, the antibody of the present invention or fragment thereof, and/or an antibody of fragment thereof that recognizes said antibody or fragment thereof is labeled with a labeling substance.

### [Examples]

### Example 1 Preparation of fragments of SARS-CoV-2 derived NPs [Design of immunogens]

Based on the sequences published on a database, the identities between the amino acid sequence of SARS-CoV-2 derived NP and the amino acid sequences of NPs derived from other coronaviruses (SARS, MERS, OC43, NL63, HKU1, 229E) were analyzed (Figure 1). As a result, it was found that the amino acid sequence of SARS-CoV-2 derived NP had about 90% amino acid sequence identity to the amino acid sequence of SARS coronavirus derived NP, had about 47% amino acid sequence identity to the amino acid sequence of MERS coronavirus derived NP, and had about 30% or less amino acid sequence identities to the amino acid sequence of NPs derived from the other coronaviruses.

Thus, it was decided to use the region of amino acids at positions 121 to 419 of SARS-CoV-2 derived NP as an immunogen, excluding the motif (FYYLGTGP), which is commonly conserved among coronaviruses.

### [Cell-free protein synthesis of fragment of SARS-CoV-2 derived NP]

Fragments of SARS-CoV-2 derived NP consisting of the amino acid sequence represented by SEQ ID NO:2 were prepared by following methods. Furthermore, SARS-CoV-2 derived NP consists of the amino acid sequence represented by SEQ ID NO:1, and the RNA sequence corresponding to said amino acid sequence is represented by SEQ ID NO:3, and the DNA sequence corresponding to said RNA sequence is represented by SEQ ID NO:4. Furthermore, said DNA sequence is codon-optimized for protein synthesis in eukaryotic cells based on the amino acid sequence.

Firstly, a DNA sequence represented by SEQ ID NO:4 was artificially synthesized, and by PCR method using said DNA sequence as a template, the region at positions 361 to 1260 of the DNA sequence represented by SEQ ID:4, which corresponds to the region at positions 121 to 419 of the amino acid sequence represented by SEQ ID NO:1 (Figure 1) was amplified. 5µL of 1µM forward primer (SEQ ID NO:5), 5µL of 1µM reverse primer (SEQ ID NO:6), 1µL of 10ng/µL DNA vector (pUC57 (GeneWiz)) into which the DNA sequence represented by SEQ ID NO:4 was inserted, 25µL of PrimeSTAR^{®} Max DNA Polymerase (Takara Bio), and 14µL of ultra pure water were mixed to prepare a reaction solution with a total volume of 50µL. PCR method was done using the thermal cycler settings shown in Table 1. By this, a DNA sequence comprising the region at positions 361 to 1260 of the DNA sequence represented by SEQ ID NO:4 was obtained. Then, by PCR method using the pEU-E01-His-TEV-MCS-N2 vector (Cell Free Science) as a template, the DNA vector was amplified according to the method using the primers shown in SEQ ID NO:7 and SEQ ID NO:8.

### [Table 1]

**Table 1. Settings for thermal cycler**

| Temperature | Time | Number of cycles |
|---|---|---|
| 98°C | 1 minutes | - |
| 98°C | 10 seconds | 30 cycles |
| 55°C | 15 seconds | |
| 72°C | 40 seconds | |
| 72°C | 2 minutes | - |

Subsequently, after the DNA sequence and DNA vectors, which were obtained from aforesaid PCR method, were purified by agarose gel electrophoresis, DNA vectors were prepared by linking DNA using In-Fusion^{®} HD Cloning Kit (Takara Bio Inc.). The obtained DNA vector was transformed into E. coli strain JM109, and the transformed E. coli was seeded on LB medium containing ampicillin. DNA vectors were prepared from E. coli after proliferation using a plasmid purification kit (Promega).

Subsequently, mRNA was transcribed from obtained DNA vectors using SP6 RNA polymerase, and fragments of SARS-CoV-2 derived NP tagged with His-tag (region at positions 121 to 419 of the amino acid sequence represented by SEQ ID NO:1 (Figure 1)) were synthesized by cell-free protein synthesis with wheat germ extract following the user instructions of WEPRO7240 kit from Cell Free Science.

### [Confirmation of expression of fragments of SARS-CoV-2 derived NP]

Expression of fragments of SARS-CoV-2 derived NP was confirmed by Western blotting method. Firstly, fragments of SARS-CoV-2 derived NP tagged with His-tag were mixed with 2×SDS sample buffer (125mM Tris-HCl, 4% SDS, 20% glycerol, 0.01% bromophenol blue, and 10% 2-mercaptoethanol), heat-treated, and subjected to polyacrylamide gels for electrophoresis. Subsequently the gels after electrophoresis were transferred to PVDF membranes. After the membranes were blocked with 2% skimmed milk solution, the membranes were allowed to react with a peroxidase-labeled anti-His tag antibody. After that, after reacting with HRP-labeled secondary antibody, peroxidase substrate solution was added to emit light, and the light emission was detected using a chemiluminescence imaging system.

As a result, as the molecular weight of fragment of SARS-CoV-2 derived NP tagged with His-tag is 33.7kDa, and a band was confirmed at the position corresponding to said molecular weight (Fig. 2A), it was able to confirm the expression of fragments of SARS-CoV-2 derived NP.

### [Purification of fragments of SARS-CoV-2 derived NP]

Fragments of SARS-CoV-2 derived NP tagged with His-tag were purified from the reaction solution of cell-free protein synthesis using Ni columns. The series of steps of transcription, cell-free protein synthesis, and purification were done using an automated synthesizer (Cell Free Science). Fragments of SARS-CoV-2 derived NP tagged with His-tag were purified over 3 times, and the first to third purified fractions were denoted as E1-E3, respectively. E1-E3 were mixed with 2×SDS sample buffer (125mM Tris-HCl, 4% SDS, 20% glycerol, 0.01% bromophenol blue, and 10% 2-mercaptoethanol), heat-treated, subjected to polyacrylamide gels for electrophoresis and dyed with Rapid CBB KANTO 3S (Kanto Chemical Co., Ltd.).

As a result, since the existing amount of contaminant proteins was reduced in E1-E3 compared to that of the solution before purification (S: soluble fraction) (Figure 2B), it was able to confirm that fragments of SARS-CoV-2 derived NP were purified.

### Example 2: Preparation of antibodies against fragments of SARS-CoV-2 derived NP

### [Immunization of mice and preparation of hybridoma cells]

300ug of purified fragments of SARS-CoV-2 derived NP were mixed with Freund's adjuvant, emulsified, and injected to BALB/c mice by subcutaneous injections in multiple times to immunize the mice. After 1 month B cells were collected from immunized mice, and fused with SP2/0 myeloma cells using polyethylene glycol solution. The fused hybridoma cells were seeded into 96-well microplates with selection medium.

### [Screening and cloning of hybridoma cells]

Subsequently, the reactivities of the monoclonal antibodies, which were prepared by each hybridoma, against fragments of SARS-CoV-2 derived NP were examined by ELISA method and AlphaScreen method.

In ELISA method, firstly, fragments of SARS-CoV-2 derived NP were fixed on an ELISA plate. After blocking the plate, culture supernatant of hybridoma was added to the plate and allowed to react, and after that peroxidase-labeled goat anti-mouse immunoglobulin antibody was added and allowed to react. After that, peroxidase substrate solution was added to develop colors, and its absorbance was measured (Figure 3A).

AlphaScreen method was performed following the manufacturer's recommended method. Firstly, biotin-labeled proteins of fragments of SARS-CoV-2 derived NP were synthesized by cell-free protein synthesis according to the procedure as described in Example 1. After that, after the synthesized biotin-labeled protein and culture supernatant of hybridoma were added to the plate and mixed, it was incubated at room temperature for 30 minutes. Then, 10µl of acceptor beads (Protein G coated) and donor beads (streptavidin coated) were added. After incubating at room temperature for further 30 minutes, measurements were done in EnVision (PerkinElmer) (Figure 3B).

By cloning the hybridomas selected in the above screening, 27 types of hybridoma cells which produce monoclonal antibodies that show high reactivities to fragments of SARS-CoV-2 derived NP were obtained.

### Example 3 Analysis of epitopes recognized by each monoclonal antibody

Each region of the amino acid sequence from fragments of SARS-CoV-2 derived NP was deleted to generate three recombinant proteins (deletion mutants), respectively and the reactivities to these proteins were compared to determine the epitope which each monoclonal antibody recognized.

To prepare each recombinant protein, firstly, using a wheat cell-free expression vector pEU-E01-His-TEV-SARS-CoV-2-NP (121-419) (SEQ ID NO:9) , which was generated in Example 1 and encodes the amino acid sequence of the amino acid sequence represented by SEQ ID NO:1, as a template, expression vectors lacking the cDNA fragments encoding the amino acid sequence regions at positions 121 to 231 (d1), 232 to 340 (d2), and 341 to 419 (d3) of the amino acid sequence represented by SEQ ID NO:1 were generated respectively following the methods of PrimeSTAR^{®} Mutagenesis Basal Kit (Takara Bio Inc.). The mRNA was synthesized from the generated DNA vector, using SP6 RNA polymerase, and each recombinant protein was synthesized by cell-free protein synthesis according to the procedure as described in Example 1, following the instructions for use of WEPRO7240 kit from Cell Free Science Ltd.

Using each obtained protein, the epitope of each monoclonal antibody was analyzed by Western blotting. Firstly, each obtained protein was mixed with 2×SDS sample buffer (125 mM Tris-HCl, 4% SDS, 20% glycerol, 0.01% bromophenol blue, and 10% 2-mercaptoethanol), heat-treated, and subjected to a polyacrylamide gel for electrophoresis. Subsequently the electrophoresed gels were transferred to PVDF membranes. After blocking the membrane with 2% skimmed milk solution, the membrane was allowed to react with each monoclonal antibody generated in Example 2. After that, after reacting with HRP-conjugated secondary antibodies, peroxidase substrate solution was added to emit light, and the light emission was detected using a chemiluminescence imaging system.

As a result, since #7 did not reacted only with d1 among 3 types of generated mutants deficient in SARS-CoV-2 derived NPs, it was found that #7 recognized the region at positions 121 to 231 of the amino acid sequence represented by SEQ ID NO:1. Since 11 clones, #2, #4, #9, #13, #40, #45, # 55, #97, #104, #111, and #128 did not react with d2 nor d3 among the generated 3 types of mutants deficient in SARS-CoV-2 derived NPs, it was found that these recognized the region at positions 232 to 419 of the amino acid sequence represented by SEQ ID NO:1. Since 15 clones, #28, #35, #47, #60, #65, #67, #86, #96, #98, #102, #103, #112, #131, #135, and #144 did not react only with d3 among the 3 types of generated mutants deficient in SARS-CoV-2 derived NPs, it was found that these recognized the region at positions 341 to 419 of the amino acid sequence represented by SEQ ID NO:1.

From the above, the generated antibodies were classified as having epitopes in the region at positions 121 to 231, 232 to 340, or 341 to 419 of the amino acid sequence represented by SEQ ID NO:1 (Figure 4A).

Subsequently, to examine more detailed recognition regions, DNA expression vectors lacking cDNA fragments encoding amino acids regions at positions 208 to 222 (d4), 203 to 222 (d5), 198 to 222 (d6), 190 to 222 (d7), 147 to 162 (d8), 398 to 419 (d9), 374 to 419 (d10), and 332 to 351 (d11) of the amino acid sequence represented by SEQ ID NO:1 respectively were generated and tested according to the method as above.

As a result, since #7 did not react with d4, d5, d6, nor d7 among 5 types of defective mutant (d4, d5, d6, d7, and d8), it was found that #7 recognized the region at positions 208 to 222 of the amino acid sequence represented by SEQ ID NO:1. Since 11 clones, #2, #4, #9, #13, #40, #45, #55, #97, #104, #111, and #128, did not react with d11 among 3 types of defective mutants (d9, d10, and d11), It was found that the 11 clones recognized the region at positions 332 to 351 of the amino acid sequence represented by SEQ ID NO:1. Since 10 clones, #35, #47, #65, #67, #86, #96, #98, #103, #112, and #131, did not react only with d10 among 3 types of defective mutants (d9, d10, and d11), it was found that the 10 clones recognized the region at positions 374 to 397 of the amino acid sequence represented by SEQ ID NO:1. It was found that since 5 clones, #28, #60, #102, #135, and #144, did not react with d9 nor d10 among 3 types of defective mutants (d9, d10, and d11), the 5 clones recognized the region at positions 398 to 419 of the amino acid sequence represented by SEQ ID NO:1.

From the above, the generated antibodies were classified as having epitopes in the regions at positions 208 to 222, 332 to 351, 374 to 397, or 398 to 419 of the amino acid sequence represented by SEQ ID NO:1 (Figure 4B).

### Example 4 Confirmation of specificity of each monoclonal antibody

Whether each monoclonal antibody specifically recognizes SARS-CoV-2 derived NPs without reacting with each one of NPs derived from other human coronaviruses (SARS, MERS, OC43, NL63, HKU1, and 229E) was examined by synthesizing recombinant proteins for each NP.

Firstly, each DNA sequence encoding each one of NPs derived from other human coronaviruses (SARS, MERS, OC43, NL63, HKU1, and 229E) was synthesized (contracted to GeneWiz) (represented by SEQ ID NO:10, 11, 12, 13, 14, and 15, respectively). Furthermore, each DNA sequence was codon-optimized for protein synthesis in eukaryotic cells based on the amino acid sequence.

Subsequently, each one of NPs derived from other human coronaviruses (SARS, MERS, OC43, NL63, HKU1, and 229E) was synthesized by cell-free protein synthesis according to the procedure as described in Example 1, respectively.

Using each protein obtained, the specificity of each monoclonal antibody was analyzed according to the Western blotting method as described in Example 3. The results are shown in Figure 5. A total of 8 antibodies that recognized SARS-CoV-2 derived NPs and did not react with each one of NPs derived from other human coronaviruses (SARS, MERS, OC43, NL63, HKU1, and 229E) were obtained (1 antibody recognizing 208 to 222 region, 3 antibodies recognizing 332 to 351 region, 3 antibodies recognizing 374 to 397 region, and 1 antibody recognizing 398 to 419 region). For example, none of #7, #9, and #98 reacted with each NPs derived from other human coronaviruses (SARS, MERS, OC43, NL63, HKU1, and 229E) but these antibodies specifically recognized SARS-CoV-2 derived NPs. On the other hand, both #128 and #144 reacted with SARS-CoV-2 derived NPs and SARS coronavirus-derived NPs, and specifically recognized SARS-CoV-2 derived NPs and SARS coronavirus-derived NPs.

### Example 5 Immunostaining of SARS-CoV-2-infected cells

Whether the monoclonal antibodies (#7, #98) generated in Example 2 react with SARS-CoV-2 infected cells was considered.

Firstly, cells derived from African green monkey kidney, VeroE6/TMPRSS2 (JCRB1819), were infected with SARS-CoV-2 provided by the National Institute of Infectious Diseases (NIID), at 37°C for 24 hours.

### [Confirmation of expression of SARS-CoV-2 derived NP]

Expression of SARS-CoV-2 derived NP was confirmed according to the Western blotting method as described in Example 1.

Firstly, lysate of aforesaid infected cells was mixed with 2×SDS sample buffer (125 mM Tris-HCl, 4% SDS, 20% glycerol, 0.01% bromophenol blue, and 10% 2-mercaptoethanol), heat-treated, and subjected to polyacrylamide gels for electrophoresis. Subsequently the electrophoresed gels were transferred to PVDF membranes. After the membranes were blocked with 2% skimmed milk solution, the membranes were allowed to react with peroxidase-labeled monoclonal antibodies (#7, #98). After that, after reacting with HRP-labeled secondary antibody, peroxidase substrate solution was added to emit light, and the light emission was detected using a chemiluminescence imaging system.

As a result, as the molecular weight of SARS-CoV-2 derived NP is 45.6kDa, and a band was confirmed at the position corresponding to said molecular weight, it was able to confirm the expression of SARS-CoV-2 derived NP(Figure 6A).

### [Immunostaining of SARS-CoV-2-infected cells]

After monoclonal antibodies (#7, #98) generated in Example 2 were added to the culture medium of aforesaid infected cells and allowed to react, and fluorescence labeled goat anti-mouse immunoglobulin antibody was added to immunostain aforesaid infected cells. After that, a fluorescence microscopy was used to image immunostained aforesaid infected cells. Furthermore, noninfected cells were used as negative controls.

As a result, a fluorescence reaction derived from binding of monoclonal antibodies (#7, #98), which were generated in Example 2, was acknowledged only for aforesaid infected cells (Figure 6B).

### Example 6 Detection of SARS-CoV-2 derived NP by ELISA method

Among monoclonal antibodies obtained in the present invention, #9 and #98, which differ in their epitopes, were used to perform ELISA. SARS-CoV-2 derived NPs (full length) were synthesized by cell-free protein synthesis based on SEQ ID NO:1 according to the procedure as described in Example 1.

#9 was diluted to 2.5ug/mL in PBS and fixed on an ELISA plate, and it was blocked with 2% skimmed milk solution. After washing the plate with PBS-T, SARS-CoV-2 derived NPs were dispensed into wells in series dilutions at 0, 1.25, 5, and 20 ng/mL and allowed to react. After washing the plate with PBS-T, it was allowed to react with peroxidase-labeled #98. After washing the plate with PBS-T, peroxidase substrate solution (KPL) was added to develop colors, and the absorbance was measured.

As a result, it was able to detect each concentration of SARS-CoV-2 derived NP (Figure 7).

The different features of the present invention described herein can be combined in various ways, and embodiments obtained by combinations as such, including combinations which are not particularly described herein, are all within the scope of the present invention. Also, a skilled person in the art understands that numerous and various modifications that do not depart from the spirit of the present invention are possible, and equivalents including such modifications are also within the scope of the present invention. Therefore, it should be understood that the embodiments described herein are illustrative only and that these are not described with the intention to limit the scope of the present invention.

## Claims

1. An antibody or fragment thereof which binds to a protein constituting a SARS-CoV-2 derived nucleocapsid, wherein an epitope which is recognized by said antibody or fragment thereof is located at positions 121 to 419 of the amino acid sequence of SEQ ID NO:1.

2. . The antibody or fragment thereof according to claim 1, wherein the epitope is located at positions 208 to 222, positions 332 to 351, positions 374 to 397, or positions 398 to 419 of the amino acid sequence of SEQ ID NO:1.

3. . The antibody or fragment thereof according to claim 1 or 2, wherein the antibody or fragment thereof specifically binds to a protein constituting a SARS-CoV-2 derived nucleocapsid.

4. . The antibody or fragment thereof according to any one of claims 1-3, wherein the antibody is a monoclonal antibody.

5. . The antibody or fragment thereof according to any one of claims 1-4, wherein the fragment is a Fab fragment, Fab/c fragment, Fv fragment, Fab' fragment or F(ab')₂ fragment.

6. . A method for detecting a protein constituting a SARS-CoV-2 derived nucleocapsid within a sample, wherein the method comprises a step of contacting the sample with an antibody or fragment thereof according to any one of claims 1-5.

7. . The method according to claim 6, wherein the sample is a biological sample.

8. . The method according to claim 6 or 7, wherein the method further comprises a step of detecting a protein constituting a SARS-CoV-2 derived nucleocapsid by at least one immunological test selected from the group consisting of ELISA, immunochromatography and filter antigen assay.

9. . A kit for detecting a protein constituting a SARS-CoV-2 derived nucleocapsid, comprising an antibody or fragment thereof according to any one of claims 1-5.

10. . The kit according to claim 9, wherein the kit is a form of immunochromatographic strip.
